# EUROPEAN PATENT SPECIFICATION

(11) **EP 2 937 087 B1**
(45) Date of publication and mention of the grant of the patent: **24.02.2021**
(21) Application number: 13865394.4
(22) Date of filing: 19.12.2013
(51) Int. Cl.: A61K 31/575, A23L 33/10, A61K 9/14, A61K 47/34, A61P 1/04, A61P 3/04, A61P 3/06, A61P 3/10, A61P 9/10, A61P 29/00, A61P 43/00

(54) **COMPOSITION AND FOOD OR DRINK**
ZUSAMMENSETZUNG UND NAHRUNGSMITTEL ODER GETRÄNK
COMPOSITION ET ALIMENT OU BOISSON

(30) Priority: 21.12.2012 JP 2012280303
(43) Date of publication of application: 28.10.2015
(73) Proprietor: SENTAN Pharma Inc., Fukuoka-shi Fukuoka 812-0027 (JP)
(72) Inventor: MASUZAKI, Hiroaki, Naha-shi Okinawa 900-0006 (JP); KOZUKA, Chisayo, Nakagami-gun Okinawa 903-0125 (JP)
(74) Representative: V.O.
(86) International application number: PCT/JP2013/084110
(87) International publication number: WO 2014/098190

(56) References cited:
- CN-A- 101 934 093
- JP-A- H09 506 109
- JP-A- 2004 262 810
- JP-A- 2004 534 721
- JP-A- 2005 503 476
- KR-B1- 101 137 785
- URACHA RUKTANONCHAI ET AL: "Effect of lipid types on physicochemical characteristics, stability and antioxidant activity of gamma-oryzanol-loaded lipid nanoparticles", JOURNAL OF MICROENCAPSULATION, vol. 26, no. 7, 1 November 2009 (2009-11-01), pages 614-626, XP055079105, ISSN: 0265-2048, DOI: 10.3109/02652040802586571
- LEE JS ET AL.: 'Gamma-oryzanol-loaded calcium pectinate microparticles reinforced with chitosan: optimization and release characteristics' COLLOIDS SURF B BIOINTERFACES. vol. 70, no. 2, 2009, pages 213 - 217, XP026012078
- REIKO NAGASAKA ET AL.: 'Komenuka Seibun y-Oryzanol no Aratana Seiri Kassei Kino to sono Riyo: NF-KB no Kassei Sogai Sayo o Kaishita Tonyobyo ya Kaiyosei Daichoen Nado no Yobo Chiryo Koka ni Kitai' KAGAKU TO SEIBUTSU vol. 47, no. 12, 2009, pages 816 - 818, XP055268658
- CHENG HH ET AL.: 'Gamma-oryzanol ameliorates insulin resistance and hyperlipidemia in rats with streptozotocin/nicotinamide-induced type 2 diabetes.' INT J VITAM NUTR RES vol. 80, no. 1, 2010, pages 45 - 53, XP008180036
- CHISAYO KOZUKA ET AL.: 'Genmai no Shinki Himan . Tonyobyo Yobo Koka no Kaimei - Shishokabu Shohotai Stress Yokusei o Kaishita Koshibo- shoku Shikosei no Teika Sayo' THE JOURNAL OF THE JAPAN DIABETIC SOCIETY vol. 55, no. SUPPLE, 25 April 2012, page 79, XP008180558
- CHISAYO KOZUKA ET AL.: 'Shishokabu Shohotai Stress Yokusei o Kaishita Genmai ni yoru Shinki Himansho Kaizen Koka' JOURNAL OF JAPAN SOCIETY FOR THE STUDY OF OBESITY vol. 17, 2011, page 165, XP008181708

## Description

### Technical Field

The present invention relates to compositions, to their medical uses, and to food or beverages comprising such compositions.

### Background Art

Brown rice diets have been known to improve insulin resistance for the better and to delay the onset of diabetes mellitus. Brown rice contains a biologically active substance, namely γ-oryzanol and mechanisms of its biological activities have been gradually uncovered. Non Patent Literature 1 shows inhibition of lipid absorption from the small intestine, promotion of insulin secretion, decreased blood glucose level, and restriction of the taste for high fat diets in obese mice that are orally administered with γ-oryzanol (320 mg/kg). Non Patent Literature 3 is directed to a study wherein γ-oryzanol was incorporated into three different types of solid lipid: wax, triglycerides, a mixture of glycerides as solid lipid nanoparticles and liquid lipid (Miglyol 812) as nanoemulsion.

### Citation List

### Non Patent Literature

Non Patent Literature 1: Kozuka, C. et al., Brown Rice and Its Component, γ-Oryzanol, Attenuate the Preference for High-Fat Diet by Decreasing Hypothalamic Endoplasmic Reticulum Stress in Mice, Diabetes, 2012, 61, 3084-93.
Non Patent Literature 2: Takeuchi, H, et al., Mucoadhesive nanoparticulate systems for peptide drug delivery, Adv. Drug Deliv. Rev., 2001, 47, 39-54.
Non Patent Literature 3: Ruktanonchai ,U. et al., Effect of lipid types on physicochemical characteristics, stability and antioxidant activity of gamma-oryzanol-loaded lipid nanoparticles, J. Microencapsul., 2009, 26(7), 614-626.

### Summary of Invention

### Technical Problem

However, in patients with obesity, dyslipidemia, diabetes mellitus, and the like, a daily dose to attain the above effect of γ-oryzanol ends up being large (e.g., the body weight of a patient is 60 kg, 19.2 g of γ-oryzanol is required). If daily oral administration of such a large amount γ-oryzanol extends for long period of time, there is a concern about adverse effects. In addition, even if the daily dose is divided to administer twice or more times, the number of times it is administered comes to be large, which makes administration management complicated. Because of these, it is not practical to orally administer a large amount of γ-oryzanol every day for a long period of time.

In the light of the above current actual circumstances, the present disclosure has been made; and an objective thereof is to provide compositions and food or beverages that allow the effect of γ-oryzanol to be attained at less dose.

### Solution to Problem

One of the reasons why a large amount of γ-oryzanol is required as just described above is thought to be a considerably low efficiency of γ-oryzanol absorption from the intestinal tract. Attention was focused on drug delivery systems (DDSs) in which drug agents are enclosed to alter the kinetics of the drug agent. In general, micro sized particle used in DDS have a property of readily adhering to the mucosa layer of the intestinal tract. Further, nanosized particles can be added with functions of penetrating into the inside of the mucosa layer, releasing drug agents, delivering to the inside of the intestinal tract (see Non Patent Literature 2). Thus far, it has not been identified *in vivo* effects of γ-oryzanol using DDS technique. In view of this, it was intensively studied whether or not the above effect can be attained even at a practical low dose of γ-oryzanol, thereby completing the present disclosure.

That is, compositions according to a first aspect of the present invention contain
γ-oryzanol and
a biocompatible particle incorporating the γ-oryzanol in the inside thereof,
wherein the biocompatible particle has a number mean particle size of 2.5 to 1,000 nm and comprises a polylactideglycolide copolymer or a polyethylene glycol modification of the polylactideglycolide copolymer.

The abovementioned biocompatible particle may contain polylactideglycolide copolymers with a number mean particle size of 2.5 to 1,000 nm or polyethylene glycol modifications thereof.
In a further aspect of the invention, there is provided the composition for use as a medicament.

In yet a further aspect of the invention, there is provided the above composition for use in improvement of insulin resistance.

In yet a further aspect of the invention, there is provided the above composition for use in suppression of endoplasmic reticulum stress.

In yet a further aspect of the invention, there is provided the above composition for use in suppression of IL-8 secretion from an intestinal epithelial cell.

In yet a further aspect of the invention, there is provided the above composition for use in treatment or prevention of a disease selected from the group consisting of obesity, dyslipidemia, impaired glucose tolerance, diabetes mellitus, arteriosclerosis, and inflammatory diseases.

The above composition may be administered to patients with a dosing interval of one day or more.

Further, the abovementioned dosing interval may be seven days or more and 14 days or less.

Food or beverages, according to a another aspect of the present invention, comprise the composition according to the first aspect of the present invention.

### Advantageous Effects of Invention

According to the present disclosure, the efficiency of γ-oryzanol absorption from the intestinal tract is increased by incorporating it in biocompatible particles and therefore the effect of γ-oryzanol can be attained at less dose.

### Brief Description of Drawings

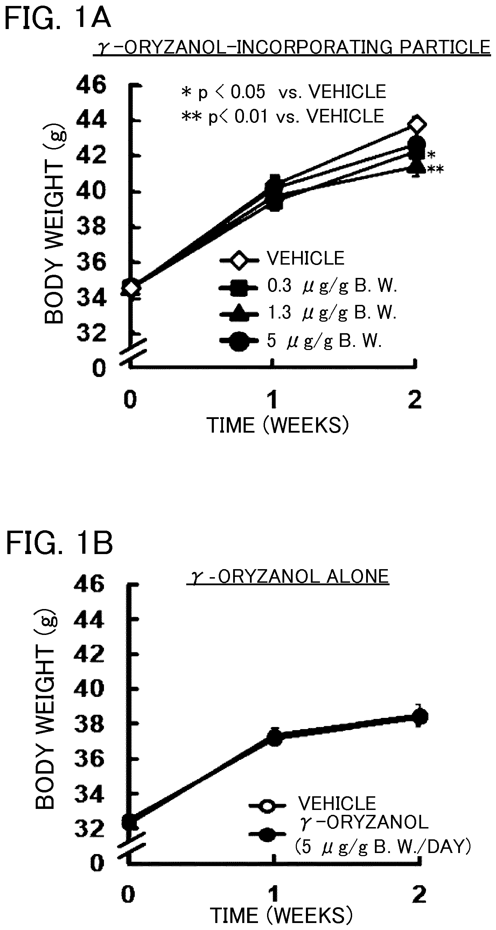

FIG. 1A shows a change of the body weight in a time-dependent manner in genetically obese mice administered with γ-oryzanol-incorporating particles; FIG. 1B shows a change of the body weight in a time-dependent manner in genetically obese mice administered with γ-oryzanol alone;

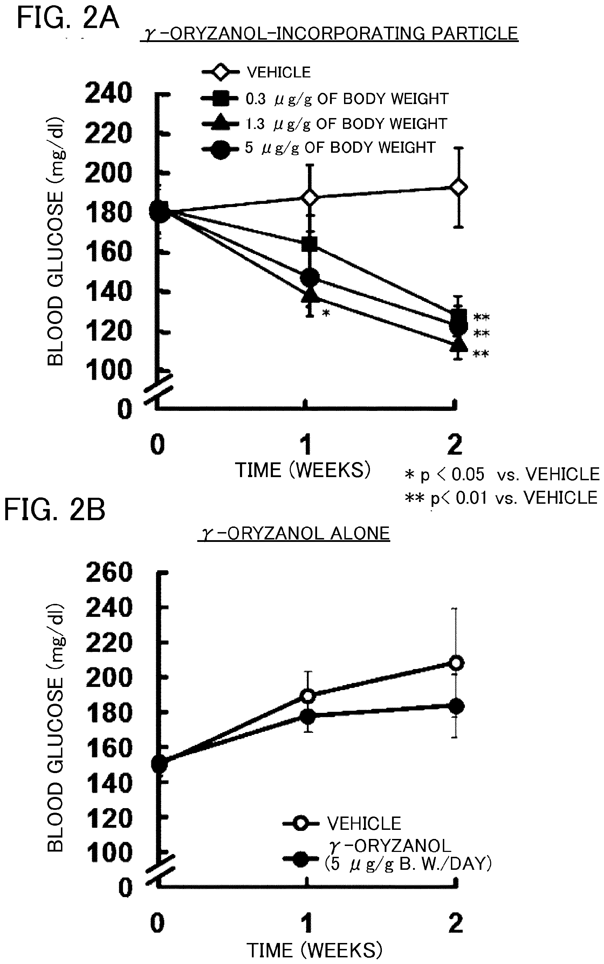

FIG. 2A shows a change of the blood glucose level in a time dependent manner in genetically obese mice administered with γ-oryzanol-incorporating particles; FIG. 2B shows a change of the blood glucose level in a time-dependent manner in genetically obese mice administered with γ-oryzanol alone;

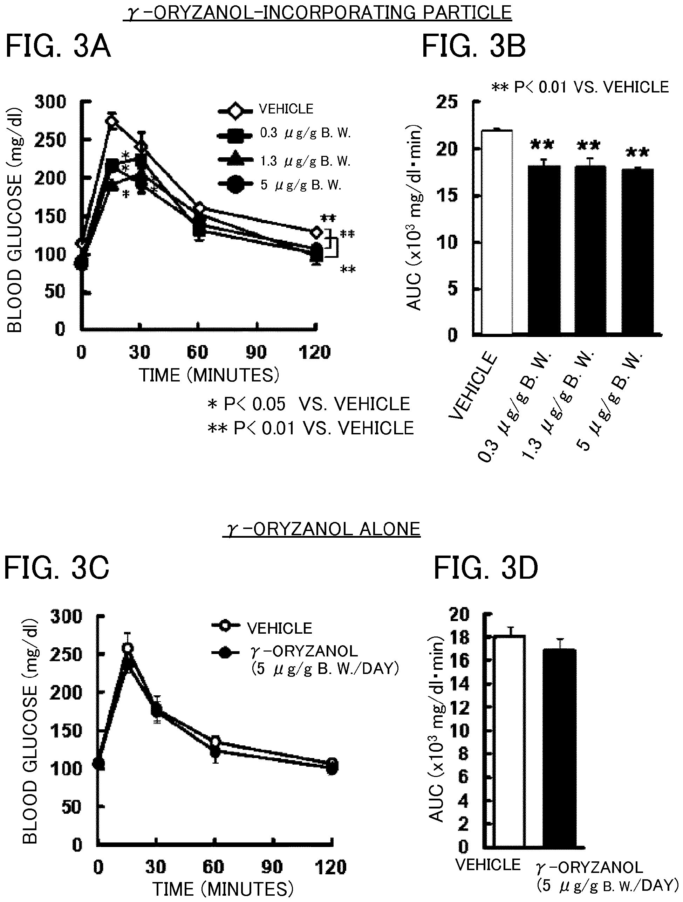

FIG. 3A (part 1) shows a change of the blood glucose level in a time-dependent manner in a glucose tolerance test for genetically obese mice administered with γ-oryzanol-incorporating particles; FIG. 3B shows the Area Under Curve (AUC) obtained from the FIG. 3A; FIG. 3C (part 1) shows a change of the blood glucose level in a time-dependent manner in a glucose tolerance test for genetically obese mice administered with y-oryzanol; FIG. 3D shows the AUC obtained from the FIG. 3C;

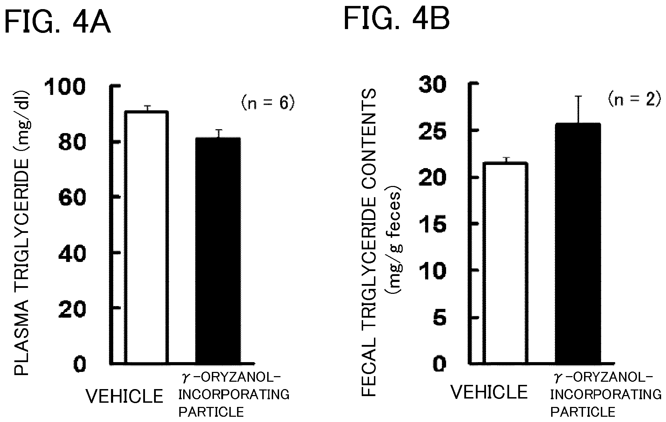

FIG. 4A shows the concentration of plasma triglyceride in genetically obese mice administered with γ-oryzanol-enclosing particles; FIG. 4B shows the concentration of lipids in feces in genetically obese mice administered with γ-oryzanol-incorporating particles;

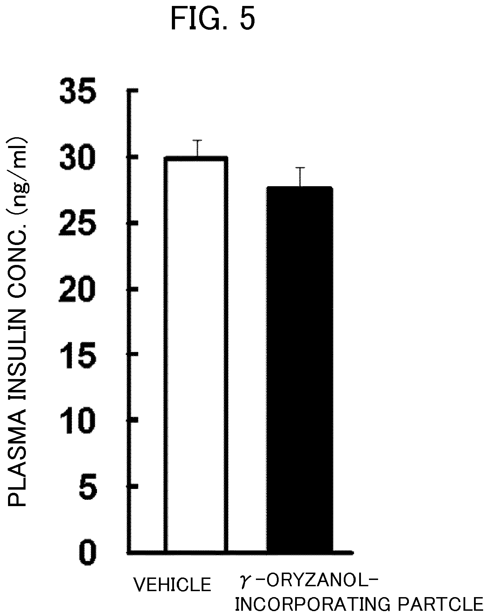

FIG. 5 shows the concentration of plasma insulin in genetically obese mice administered with γ-oryzanol-enclosing particles;

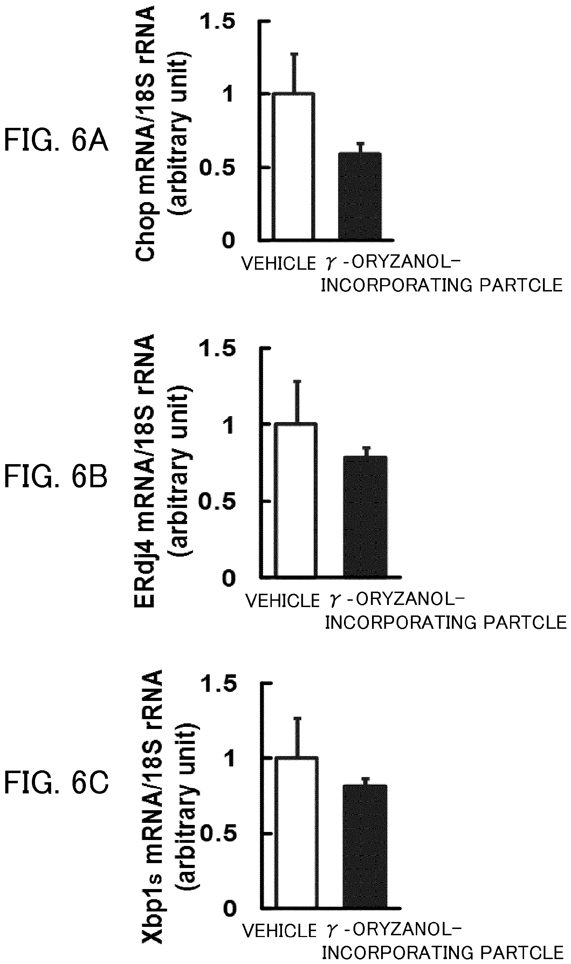

FIG. 6A, FIG. 6B and FIG. 6C show the expression level of endoplasmic reticulum (ER) stress marker genes in genetically obese mice administered with γ-oryzanol-incorporating particles; FIG. 6A shows that the expression level of Chop; FIG. 6B shows that the expression level of ERdj4; FIG. 6C shows that the expression level of Xbpls;

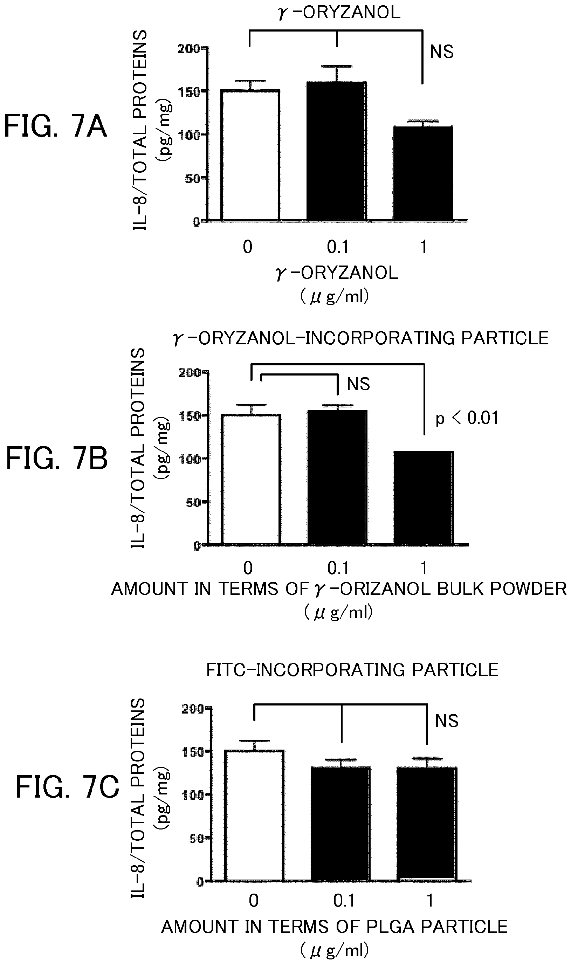

FIG. 7A, FIG. 7B and FIG. 7C show the amount of IL-8 secreted from TNF-α-stimulated intestinal epithelial cells; FIG. 7A shows the case in which the cells were pretreated with γ-oryzanol alone; FIG. 7B shows the case in which the cells were pretreated with γ-oryzanol-incorporating particles; FIG. 7C shows the case in which the cells were pretreated with FITC-incorporating particles that were used as a control for PLGA particles;

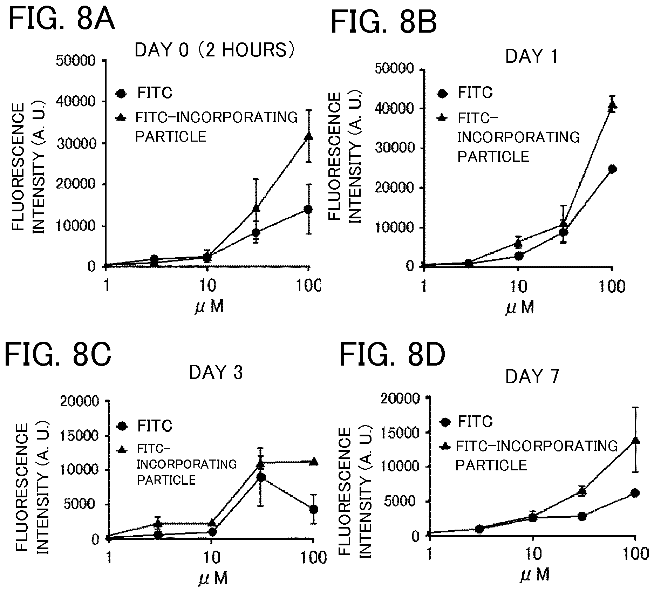

FIG. 8A, FIG. 8B, FIG. 8C and FIG. 8D show the amount of FITC taken up by intestinal epithelial cells, which amount is expressed as the fluorescence intensity analyzed by using a confocal laser scanning microscope; FIG. 8A shows the fluorescence intensity on Day 0 after the addition of FITC-incorporating particles; FIG. 8B shows the fluorescence intensity one day after the addition of FITC-incorporating particles; FIG. 8C shows the fluorescence intensity three days after the addition of FITC-incorporating particles; FIG. 8D shows the fluorescence intensity seven days after the addition of FTTC-enclosing particles;

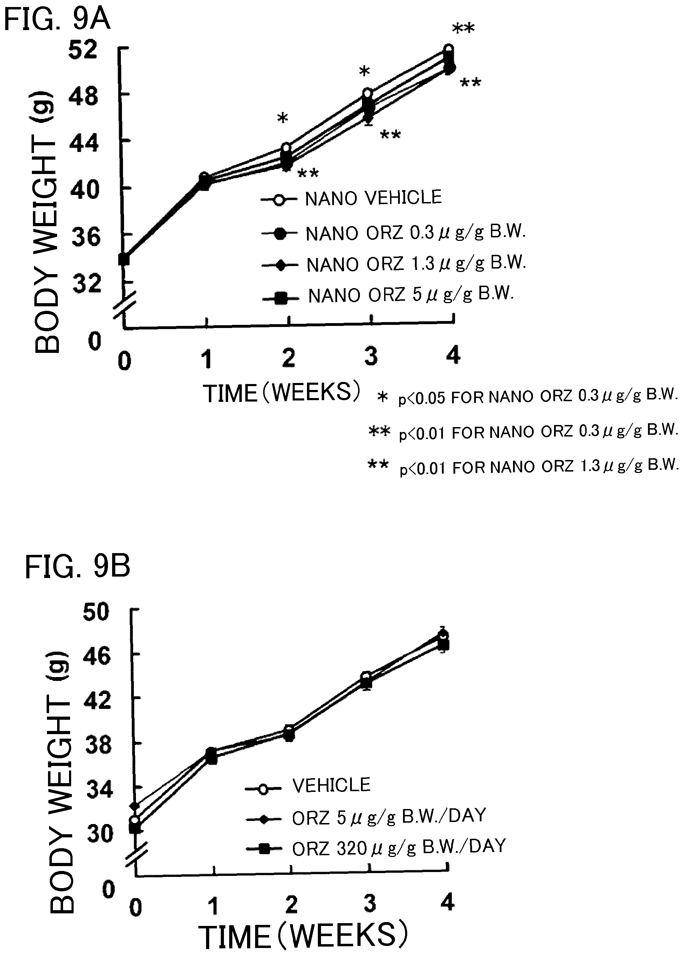

FIG. 9A shows a change of the body weight in a time-dependent manner for four weeks in genetically obese mice administered with γ-oryzanol-incorporating particles; FIG. 9B shows a change of the body weight in a time-dependent manner for four weeks in genetically obese mice administered with γ-oryzanol alone;

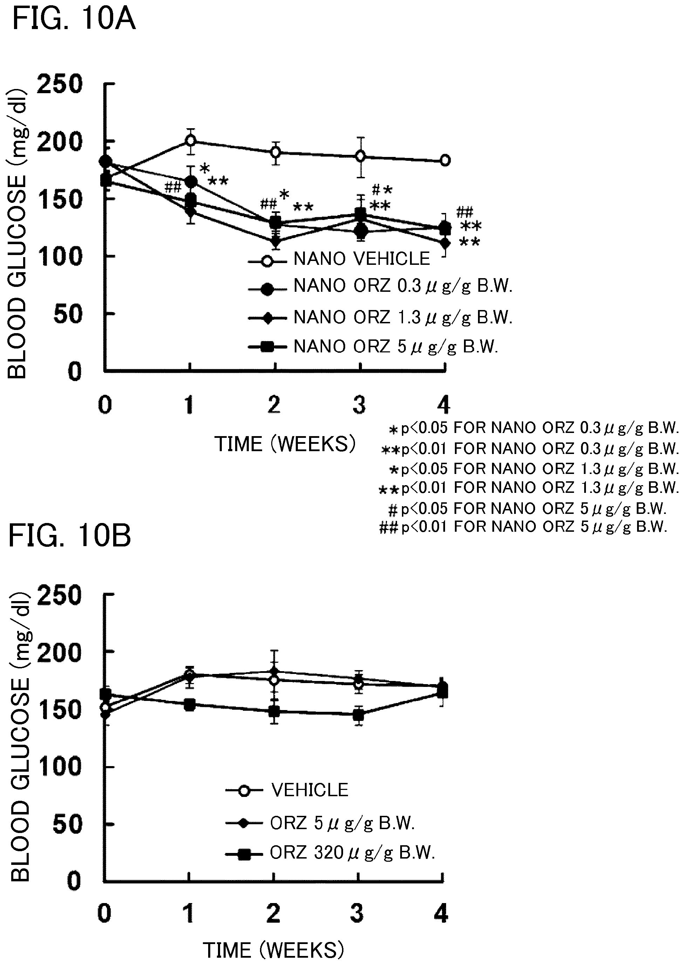

FIG. 10A shows a change of the blood glucose level in a time-dependent manner for four weeks in genetically obese mice administered with γ-oryzanol-incorporating particles; FIG. 10B shows a change of the blood glucose level with time for four weeks in genetically obese mice administered with γ-oryzanol alone;

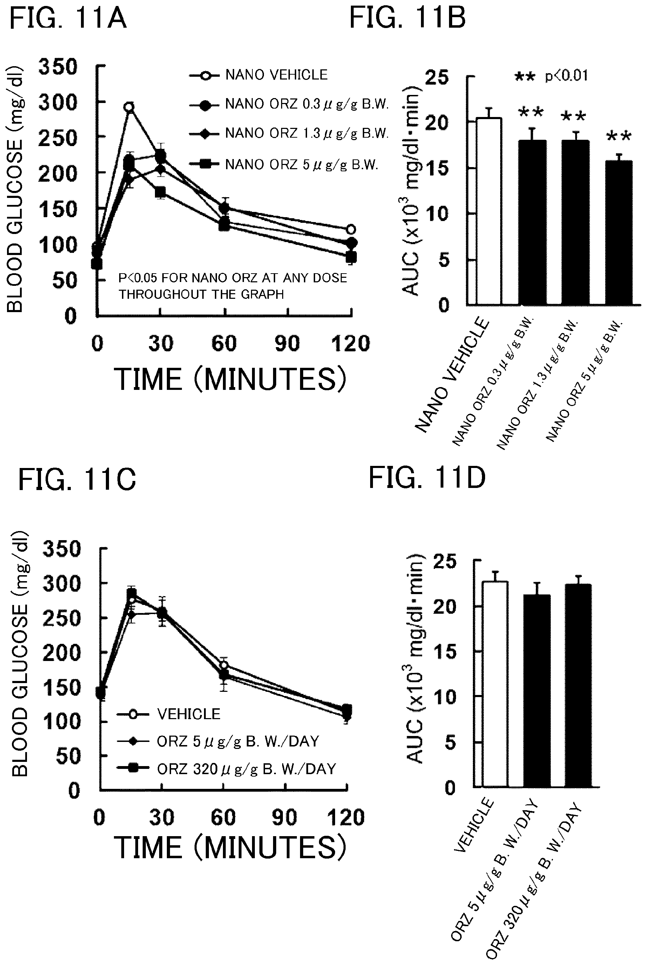

FIG. 11A (part 2) shows a change of the blood glucose level in a time-dependent manner in a glucose tolerance test for genetically obese mice administered with γ-oryzanol-incorporating particles; FIG. 11B shows the AUC obtained from the FIG. 11A; FIG. 11C (part 2) shows a change of the blood glucose level in a time-dependent manner in a glucose tolerance test for genetically obese mice administered with γ-oryzanol alone; FIG. 11D shows the AUC obtained from FIG. 11C;

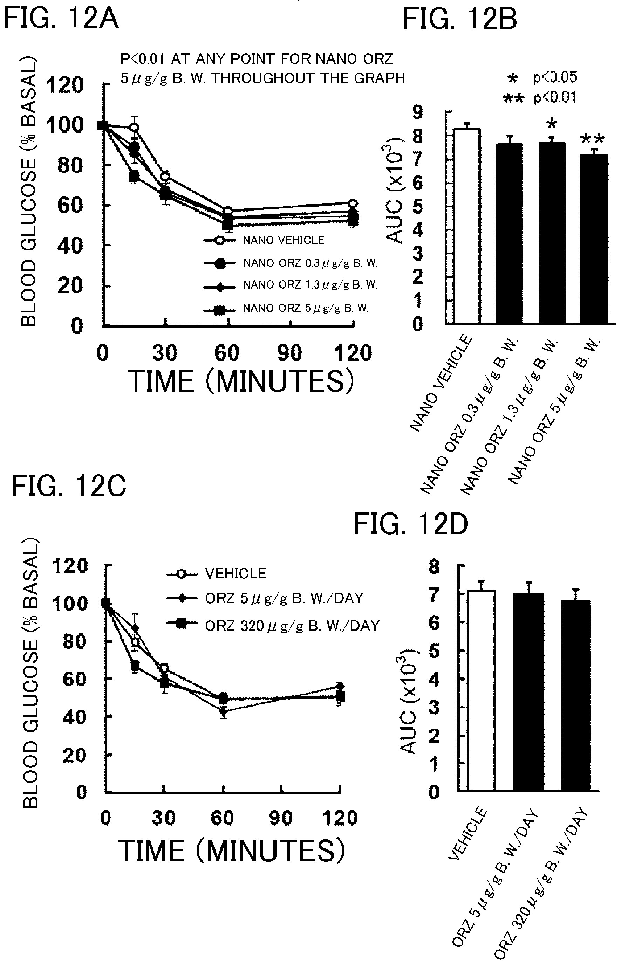

FIG. 12A shows a change of the blood glucose level in a time-dependent manner in an insulin tolerance test for genetically obese mice administered with γ-oryzanol-incorporating particles; FIG. 12B shows the AUC obtained from the FIG. 12A; FIG. 12C shows a change of the blood glucose level in a time-dependent manner in an insulin tolerance test for genetically obese mice administered with γ-oryzanol alone; FIG. 12D shows the AUC obtained from FIG. 12C;

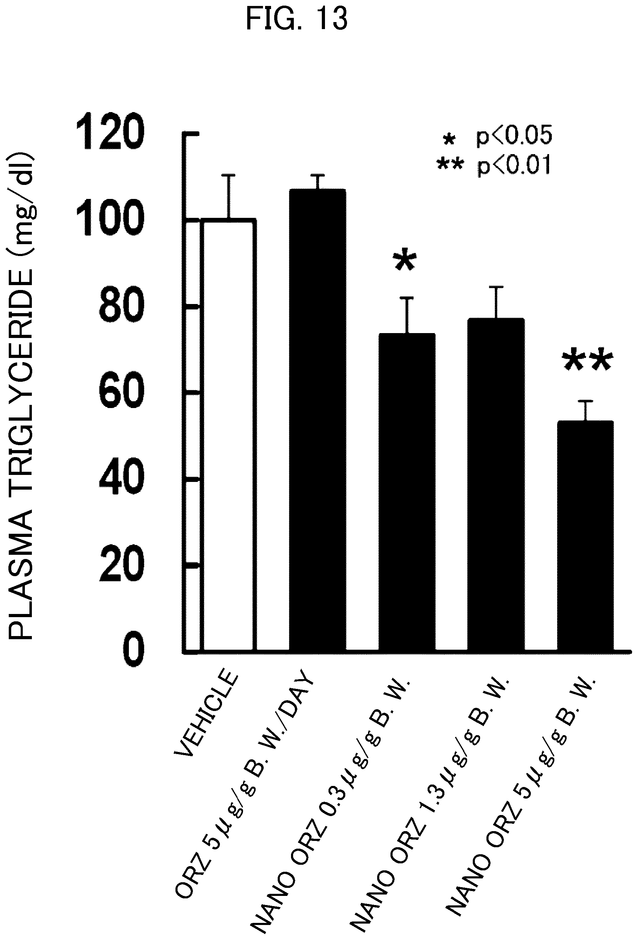

FIG. 13 shows the concentration of plasma triglyceride in genetically obese mice administered with γ-oryzanol-incorporating particles or γ-oryzanol alone;

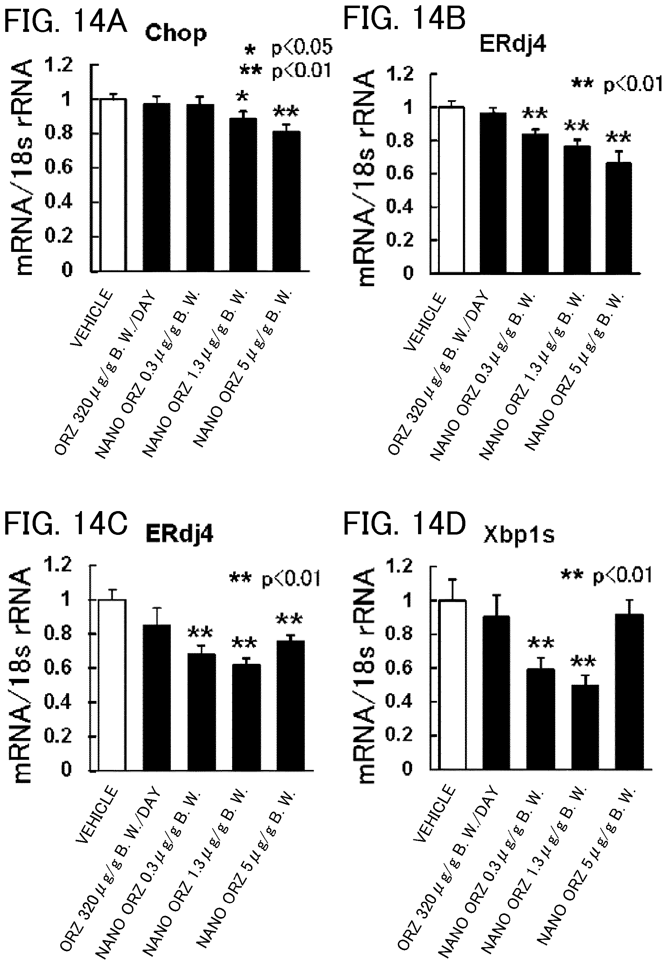

FIG. 14A, FIG. 14B, FIG. 14C and FIG. 14D show the expression level of ER stress marker genes in genetically obese mice administered with γ-oryzanol-incorporating particles or γ-oryzanol alone; FIG. 14A shows the expression level of Chop in the hypothalamus; FIG. 14B shows the expression level of ERdj4 in the hypothalamus; FIG. 14C shows the expression level of ERdj4 in the islets of Langerhans; FIG. 14D shows the expression level of Xbp1s in the islets of Langerhans;

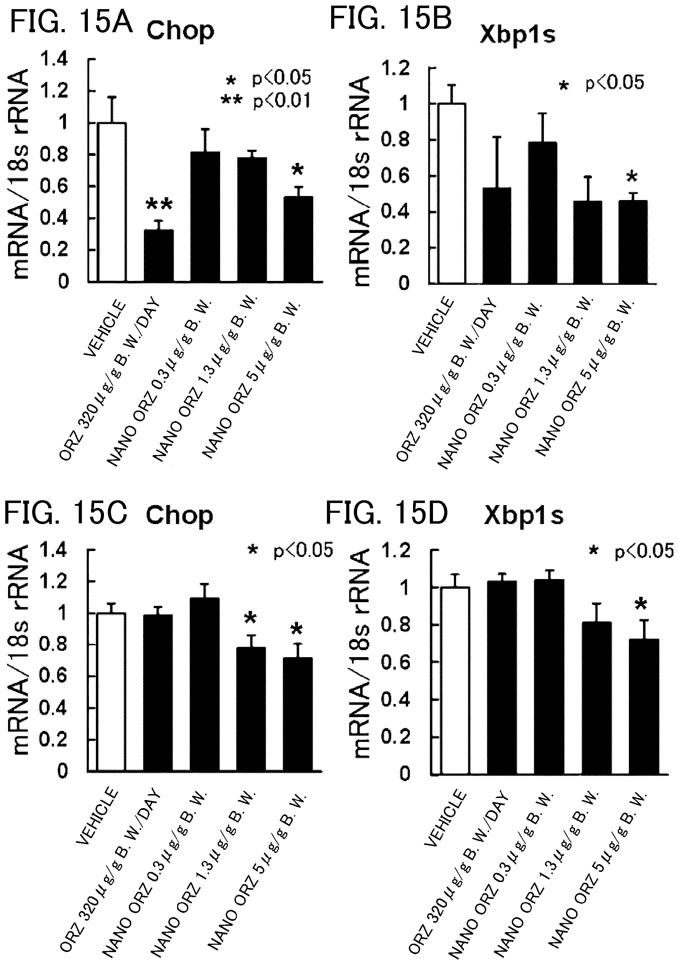

FIG. 15A, FIG. 15B, FIG. 15C and FIG. 15D show the expression level of ER stress marker genes in genetically obese mice administered with γ-oryzanol-enclosing particles or y-oryzanol; FIG. 15A shows the expression level of Chop in the fatty tissue of the mesentery; FIG. 15B shows the expression level of Xbp1s in the fatty tissue of the mesentery; FIG. 15C shows the expression level of Chop in the liver; FIG. 15D shows the expression level of Xbp1s in the liver; and

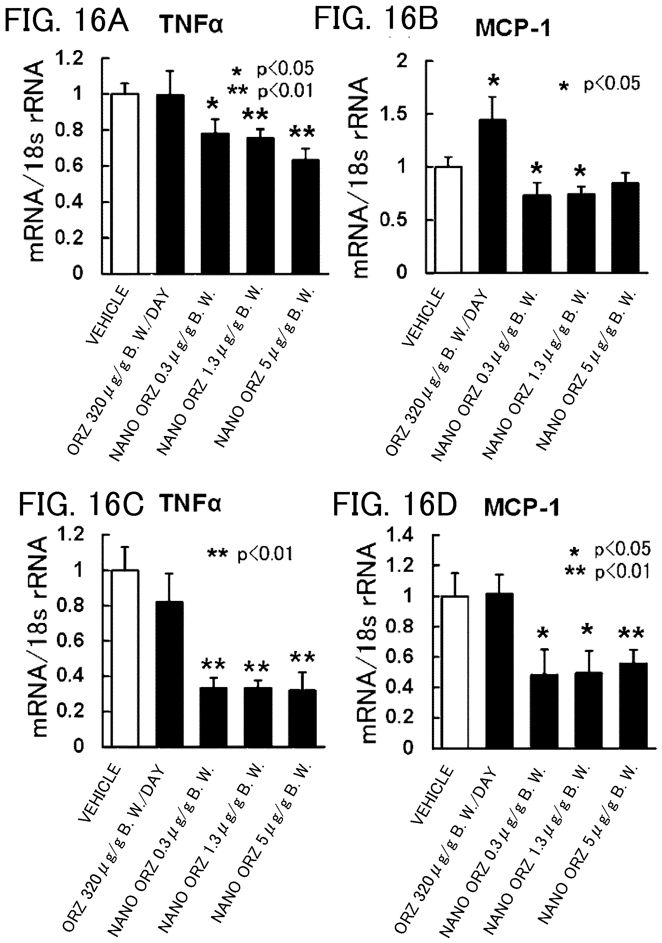

FIG. 16A, FIG. 16B, FIG. 16C and FIG. 16D show the expression level of inflammatory cytokine and chemokine genes in genetically obese mice administered with γ-oryzanol-enclosing particles or γ-oryzanol; FIG. 16A shows the expression level of TNF-α in epididymal adipose tissue; FIG. 16B shows the expression level of MCP-1 in epididymal adipose tissue; FIG. 16C shows the expression level of TNF-α in the subcutaneous adipose tissue; FIG. 16D shows the expression level of MCP-1 in the subcutaneous adipose tissue.

### Description of Embodiments

The embodiments according to the present invention will be described.

### (Embodiment 1)

First, embodiment 1 will be described. Compositions according to the present embodiment contain γ-oryzanol and a biocompatible particle incorporating the γ-oryzanol in the inside thereof, wherein the biocompatible particle has a number mean particle size of 2.5 to 1,000 nm and comprises a polylactideglycolide copolymer or a polyethylene glycol modification of the polylactideglycolide copolymer.

γ-Oryzanol is a bioactive substance which is included in lipids of rice bran or the like. γ-Oryzanol is a compound in which a sterol or triterpene alcohol is coupled with ferulic acid via an ester bond or a mixture thereof. γ-Oryzanol can be extracted mainly from rice bran oil or rice germ oil. In addition, commercially available γ-oryzanol can also be used (manufactured by Wako Pure Chemical Industries, Ltd.).

Biocompatible particles that incorporate γ-oryzanol inside thereof can be produced from biocompatible polymers. The biocompatible polymers vary in their average chain length, leading to difference in inherent viscosity and polymer characteristics. Polymers used in the present embodiment are preferably those that possess biocompatibility, being less stimulative and less toxic to organisms and that are biodegradable, being broken down after administered to be metabolized. Examples of the polymer that is biodegradable include polymers, such as polyhydroxybutyrate or polyhydroxyvalerate; natural polymers, such as collagen, cellulose acetate, bacterial cellulose, high amylose corn starch, starch, or chitosan.

The biocompatible particle obtained from the biocompatible polymer preferably releases the incorporating γ-oryzanol in a sustained, that is, controlled fashion. Because of this, those with a molecular weight of 5,000 to 200,000 or a molecular weight 15,000 to 25,000 are, for example, preferred as the biocompatible polymer.

Biocompatible particles can be produced from biocompatible polymers, for example, biocompatible polyesters. The biocompatible polyester is a polyester synthesized by polymerizing one or more kinds of monomers selected from, for example, D,L-lactide, D-lactide, L-lactide, D,L-lactic acid, D-lactic acid, L-lactic acid, glycolide, glycolic acid, ε-caprolactone, ε-hydroxyhexanoic acid, γ-butyrolactone, γ-hydroxybutyric acid, δ-valerolactone, δ-hydroxyvaleric acid, hydroxybutyric acid, malic acid, and the like. The biocompatible polymer is preferably polylactic acid, polyglycolic acid, lactic acid· glycolic acid copolymers, or lactic acid ·aspartic acid copolymers, and, in particular, poly(lactic-co-glycolic acid) (PLGA) or polyethylene glycol/chitosan modified-PLGA (PEG/CS-PLGA).

PLGA as a biocompatible polymer is a copolymer comprising lactic acid or lactide and glycolic acid or glycolide at a ratio of, for example, 1:99 to 99:1, and preferably 3:1. PLGA may be synthesized from any monomers by a common method; or commercially available PLGA may be used. Examples of commercially available PLGA include PLGA7520 (lactic acid: glycolic acid = 75:25, weight average molecular weight 20,000, manufactured by Wako Pure Chemical Industries, Ltd.). PLGA whose content of lactic acid and glycolic acid is 25% by weight to 65% by weight is amorphous and is preferred in that such a PLGA is soluble in organic solvents, such as acetone.

In cases where the above biocompatible particle is produced from PLGA, the biocompatible particle may be produced so as to contain PLGA with a particle size of less than 1,000 nm, for example, 2.5 to 1,000 nm, preferably 5 to 800 nm, more preferably 25 to 500 nm, still more preferably about 50 to 300 nm, and most preferably 100 to 250 nm. The particle size can be measured by a sieving method, a precipitation method, a microscope method, a light scattering method, a laser diffraction/scattering method, an electrical resistance test, or the like. The particle size can be expressed in Stokes radius, equivalent circular diameter, or equivalent spherical diameter depending on a method for measurement. In addition, the particle size may be number mean particle size, volume mean particle size, area mean particle size, or the like, which represents a mean obtained by subjecting plural particles to the measurement. For example, the number mean particle size is a mean particle size calculated from number distribution or the like based on measurement by a laser diffraction/scattering method or the like. To be specific, when a cumulative curve is determined with the total volume of the population of powders as 100%, 50% diameter (D50) which a particle size at a point where the cumulative curve reaches 50% may be considered to be as the mean particle size.

The biocompatible particle produced with PLGA is easy to be retained in the mucosa layer of the intestinal tract (the small intestine) or the intestinal wall, and is preferred in that the incorporating γ-oryzanol is sustainedly released in the mucosa layer of the intestinal tract, the intestinal wall, or the like.

Further, polyethylene glycol (PEG) modifications of PLGA may be used in the above production of the biocompatible particle. When the surface of PLGA is modified with PEG, the stability in the blood is improved. In this respect, the PEG modification is preferred.

The above biocompatible particle can be produced by any method as long as the method is a method capable of processing biocompatible polymers and γ-oryzanol into particles with a number mean particle size of 2.5 to 1,000 nm, preferably 5 to 800 nm, more preferably 25 to 500 nm, still more preferably about 50 to 300 nm, and most preferably 100 to 250 nm, when measured by, for example, a laser diffraction/scattering method or the like. The biocompatible particle can be produced by, for example, spherical crystallization technique. The spherical crystallization technique is a method of making particles wherein crystals precipitated and deposited during crystallization operation are turned spherical. According to the spherical crystallization technique, produced compounds can be processed while the physical property of the compound is being controlled. Among the spherical crystallization technique, there is, for example, an emulsion solvent diffusion method (hereinafter referred to simply as "ESD method").

In the ESD method, two types of solvent are used: a good solvent which dissolves biocompatible polymers and a poor solvent which does not dissolve biocompatible polymers. For the good solvent, organic solvents that dissolve biocompatible polymers and are miscible with the poor solvent are used. The kind of the good solvent and poor solvent is determined depending on the kind or the like of substances that are enclosed; and the kind of the good solvent and poor solvent is not particularly restricted. Because the biocompatible particles according to the present embodiment are used as raw materials of compositions that act mainly on the human body, it is preferred that those having a high safety to the human body and exhibiting less environmental burden be used.

Examples of the poor solvent include water; and surfactants may be added to water. As the surfactant, an aqueous polyvinyl alcohol solution is for example preferred. As surfactants other than polyvinyl alcohol, examples include lecithin, hydroxymethyl cellulose, and hydroxypropyl cellulose. It is to be noted that, when polyvinyl alcohol remains, polyvinyl alcohol may be removed by centrifugation or the like after the evaporation of the solvent.

Examples of the good solvent include halogenated alkanes, acetone, methanol, ethanol, ethyl acetate, diethyl ether, cyclohexane, benzene, and toluene, which are organic solvents with a low boiling point and poor water solubility. Preferably, acetone which exhibits less adverse effects on the environment or the human body is used solely or as a mixed solution with ethanol.

In the ESD method, a biocompatible polymer is first dissolved in a good solvent; and in order for the biocompatible polymer not to precipitate, a γ-oryzanol solution is thereafter added to and mixed with the good solvent. When the mixed solution containing the biocompatible polymer and the γ-oryzanol is, while stirred, dropped to a poor solvent, the good solvent in the mixed solution rapidly diffuses and migrates into the poor solvent. As a result, the good solvent is emulsified in the poor solvent to form emulsion droplets of the good solvent with a diameter of about several µm. Further, because the organic solvent continuously diffuses from the inside of the emulsion to the poor solvent due to mutual diffusion between the good solvent and the poor solvent, the solubility of the biocompatible polymer and γ-oryzanol in the emulsion droplet decreases. Eventually, biocompatible particles, which are spherical crystal particles incorporating γ-oryzanol, are generated. Thereafter, the organic solvent, which is the good solvent, is separated with centrifugation and distilled off under reduced pressure to obtain biocompatible particle powders. The obtained powders are used as they are or are, as necessary, subjected to composite formation via freeze-drying or the like to yield aggregation particles capable of being redispersed. The composite particles are filled in a vessel as γ-oryzanol-incorporating particles. In the thus obtained γ-oryzanol-incorporating particle, γ-oryzanol is preferably incorporated in 0.1 to 99%(w/v), more preferably 0.1 to 30%(w/v), still more preferably 1 to 10%(w/v), and in particular preferably 3 to 5%(w/v).

Because the obtained γ-oryzanol- incorporating particle is substantially spherical in the above spherical crystallization technique, there is no need to consider a problem concerning residual catalysts or starting compounds. In addition, according to the spherical crystallization technique, it is possible to readily form the γ-oryzanol-incorporating particles with less variation of particle size.

It is to be noted that, for the purpose of increasing the incorporating percentage of γ-oryzanol inside of the biocompatible particle, cationic polymers may be added to the poor solvent. In cases where the cationic polymer is added in the poor solvent, it is thought that the cationic polymer adsorbed on the surface of the biocompatible particle interacts with the γ-oryzanol present on the surface of the emulsion droplet, which allows for inhibition of γ-oryzanol leakage into the poor solvent.

Examples of the cationic polymer include chitosan and chitosan derivatives; cationized cellulose in which plural cationic groups are bound to cellulose; polyamino compounds, such as polyethyleneimine, polyvinylamine, or polyallylamine; poly amino acids, such as polyornithine or polylysine; polyvinyl imidazole, polyvinyl pyridinium chloride, alkylamino methacrylate quaternary salt polymer (DAM), and alkylamino methacrylate quaternary salt acrylamide copolymer (DAA). In particular, chitosan or derivatives thereof are suitably used.

Chitosan is a natural polymer in which a large number of glucosamines are linked, which glucosamine is a type of sugar with an amino group; and has characteristics of emulsion stability, shape retainability, biodegradability, biocompatibility, antimicrobial activities, and the like; and thus widely used as raw materials of cosmetics, food products, clothing, pharmaceutical products, and the like. Addition of this chitosan to the poor solvent enables the production of the γ-oryzanol-enclosing particles that exhibit no adverse effects to organisms and are highly safe.

The γ-oryzanol-incorporating particle obtained as described above can be subjected to composite formation to yield aggregate particles (nanocomposites) capable of being redispersed upon powderization by freeze-drying or the like. At that time, it is preferred that an organic or inorganic substance be turned into a composite capable of being redispersed and dried together with the γ-oryzanol-incorporating particle. For example, by using sugar alcohol or sucrose, variation of an incorporating percentage can be effectively prevented and, at the same time, the sugar alcohol or the like can serve as an excipient to increase ease of handling of the γ-oryzanol-incorporating particle. Examples of the sugar alcohol include mannitol, trehalose, sorbitol, erythritol, maltitose, and xylitose. Of these, trehalose is in particular preferred.

This composite formation allows γ-oryzanol-incorporating particles to become easy-to-handle aggregation particles. When used, the aggregation particles are dissociated upon contact with water, the γ-oryzanol-enclosing particles exhibit their characteristics, such as high reactivity. It is to be noted that, in place of a freeze-drying method, a fluidized bed dry granulation method using, for example, Agromaster AGM (manufactured by Hosokawa Micron Corporation) can be employed to form composites to achieve integration in a state where redispersion is feasible.

Compositions containing the γ-oryzanol-enclosing particle according to the present embodiment are produced by a known method and contain, as an active component, γ-oryzanol-incorporating particles at about 0.1% to 99%, about 1% to about 50%, and preferably 1 to 20% (% refers to % by weight).

Compositions containing the γ-oryzanol-incorporating particle according to the present embodiment can be used, in their medicine application, as injections, rectal suppositories, vaginal suppositories, transnasal absorption preparations, transdermal absorption preparations, pulmonary absorption preparations, intraoral absorption preparations, or the like. In particular, it is preferred that the composition containing γ-oryzanol-incorporating particle be used as an oral administration preparation because the composition exhibits a high absorption efficiency in the intestinal tract. In this case, such a composition may be formulated with, for example, a pharmaceutically acceptable carrier to be used as a combination drug product. The pharmaceutically acceptable carrier is various organic carrier substances or inorganic carrier substances that are used as formulation materials. The pharmaceutically acceptable carrier is formulated in therapeutic agents for oxidative stress diseases, for example, as an excipient, a lubricant, a binder, a disintegrant in the case of solid preparations; or as a solvent, a solubilizing agent, a suspending agent, an isotonic agent, a buffering agent, a soothing agent in the case of liquid preparations. In addition, additives, such as antiseptics, antioxidants, coloring agents, or sweeteners, can be used as necessary.

The excipient is, for example, lactose, white sugar, D-mannitol, starch, crystalline cellulose, light anhydrous silicic acid, or the like. The lubricant is, for example, magnesium stearate, calcium stearate, talc, colloidal silica, or the like. The binder is, for example, crystalline cellulose, white sugar, D-mannitol, dextrin, hydroxypropyl cellulose, hydroxy propyl methylcellulose, polyvinylpyrrolidone, or the like. The disintegrant is, for example, starch, carboxymethyl cellulose, carboxymethyl cellulose calcium, croscarmellose sodium, carboxymethyl cellulose sodium, or the like.

The solvent is, for example, water for injection, alcohol, propylene glycol, macrogol, or the like. The solubilizing agent is, for example, polyethylene glycol, propylene glycol, D-mannitol, benzyl benzoate, ethanol, tris aminomethane, cholesterol, triethanolamine, sodium carbonate, sodium citrate, or the like. The suspending agent is a surfactant, a hydrophilic polymer, or the like, for example stearyltriethanolamine, sodium lauryl sulfate, laurylamino propionic acid, lecithin , benzalkonium chloride, benzethonium chloride, glyceryl monostearate , polyvinyl alcohol, polyvinylpyrrolidone, sodium carboxymethyl cellulose , methylcellulose , hydroxymethyl cellulose , hydroxyethyl cellulose , hydroxypropyl cellulose, or the like.

The isotonic agent is, for example, sodium chloride, glycerin, D-mannitol, or the like. The buffering agent is, for example, phosphate buffer, acetate buffer, carbonate buffer, citrate buffer, or the like. The soothing agent is, for example, benzyl alcohol, or the like. The antiseptic is, for example, para-hydroxybenzonates, chlorobutanol, benzyl alcohol, phenethyl alcohol, dehydroacetic acid, sorbic acid, or the like. The antioxidant is, for example, sulfite, ascorbic acid, or the like.

As shown in Examples 2 and 5 below, it is suitable that compositions containing the γ-oryzanol-incorporating particle according to the present embodiment are used for improvement of insulin resistance (insulin-sensitizing agents). Further, in addition to having effects of suppressing an increase in blood glucose levels, weight gain, and lipid absorption, such compositions are thought to have an antiatherosclerotic effect because the compositions decrease the concentration of triglyceride in the plasma. In the light of these, it is suitable that such compositions are used for treatment or prevention of diseases selected from, for example, obesity, dyslipidemia, impaired glucose tolerance, diabetes mellitus, and arteriosclerosis. It is to be noted that because such compositions suppress ER stress, the compositions may be used for suppression of the ER stress, or treatment or prevention of diseases associated with the ER stress (ER stress suppressants). In particular, as shown in Example 5 below, such compositions significantly suppress the ER stress in the hypothalamus. Such compositions relieve addiction to high fat diet even at low dose because the ER stress in the hypothalamus which controls eating is involved in preference for high fat diet. Also in this respect, such compositions are suitable for treatment or prevention of obesity, diabetes mellitus, or the like. Besides, compositions containing the γ-oryzanol-incorporating particle may be used for treatment or prevention of diseases, such as coronary artery disease, hypertension, metabolic syndrome, neurodegenerating disease, and bipolar disorder, which are diseases associated with the ER stress.

As shown in Example 3 below, compositions containing the γ-oryzanol-incorporating particle according to the present embodiment suppress IL-8 secretion from intestinal epithelial cells. It is thus suitable that such compositions are used for suppression of the IL-8 secretion from intestinal epithelial cells (IL-8 secretion suppressants). When the secretion of IL-8 increases, immune cells such as neutrophils are recruited. The immune cells release a large amount of inflammatory cytokines and thereby inflammatory diseases further advance. Because of this, compositions containing γ-oryzanol-incorporating particle may be used for treatment or prevention of inflammatory diseases in the intestinal epithelia, such as ulcerative colitis or Crohn's disease. As shown in Example 5, such compositions have been also shown to suppress expression of inflammatory cytokines and chemokines not only in the intestinal epithelia but also the fatty tissue of the epididymis and the subcutaneous fatty tissue. Because of this, it is suitable that such compositions are sued for treatment or prevention of inflammatory diseases.

A dose of the composition containing the γ-oryzanol-incorporating particle according to the present embodiment is determined as appropriate in accordance with subjects' gender, age, body weight, symptoms, or the like. Such a composition is administered at a therapeutically effective amount in terms of γ-oryzanol. An effective amount refers to an amount of γ-oryzanol that is necessary to achieve a desired result and an amount necessary to result in delay in progression, inhibition, prevention, reversal, or cure of conditions subjected to therapy or treatment. The dose of such a composition is typically about 0.01 mg/kg to about 1,000 mg/kg, preferably about 0.1 mg/kg to about 200 mg/kg, more preferably about 0.2 mg/kg to about 20 mg/kg; and is administered once a day or dividedly in twice or more times. In the case where such a composition is dividedly administered, the composition is preferably administered once to four times per day. Further, such a composition may be administered at various kinds of dosing frequency, such as every day, every other day, once a one week, every other week, or once a month. It is preferred that the dosing frequency is readily determined by medical doctors or the like. It is to be noted that an amount out of the above range can be used as necessary.

As shown in Example 4 below, the uptake of particle that incorporates γ-oryzanol according to the present embodiment, for example, the PLGA particle into intestinal epithelial cells is maintained over a long period of time. Because of this, as shown in Examples 2 and 5 below, weekly or biweekly administration of the composition containing the γ-oryzanol-incorporating particle according to the present embodiment is able to achieve effects including improvement of insulin resistance, suppression of blood glucose level, weight gain, and lipid absorption, decrease in the concentration of triglyceride in the plasma, suppression of ER stress, and inhibition of inflammation. Thus, such a composition containing γ-oryzanol-incorporating particle may be administered to patients with a dosing interval of one day or more. Such a dosing interval may be seven days or more and 14 days or less.

As described in detail above, the composition according to the present embodiment has an increased absorption efficiency of γ-oryzanol from the intestinal tract by incorporating γ-oryzanol in the biocompatible particle and is therefore able to attain the effect of γ-oryzanol at less dose.

Further, because the composition according to the present embodiment is able to attain sufficient effects at lower dose, as compared with cases where γ-oryzanol is not incorporated in the biocompatible particle, the number of administration can be reduced. In addition, because the administration is easy and adverse effects ascribed to a large administration amount can be prevented, the safety can be increased.

It is to be noted that, in the present embodiment, the biocompatible particle for incorporating γ-oryzanol may contain PLGA with a number mean particle size of 2.5 to 1,000 nm or PEG modification thereof as well. PLGA is suitable for administration to the human body because PLGA exhibits biocompatibility of being less stimulative and less toxic to organisms and biodegradability of being broken down after administration to be metabolized. Further, PEG modifications of PLGA have improved stability in the blood and are therefore useful for optimizing pharmacokinetics profile such as metabolism absorption stability. Further, it is thought that PLGA and PEG modifications of PLGA that enclose γ-oryzanol retain in the mucosa layer of the intestinal tract and the intestinal wall and gradually release the γ-oryzanol enclosed. Because of this, the effect of γ-oryzanol can be attained at less dose over a relatively long period of time.

Further, also allowed are methods of treating or preventing obesity, dyslipidemia, impaired glucose tolerance, diabetes mellitus, arteriosclerosis, and inflammatory diseases comprising administering the compositions according to the present embodiment to patients. Further, the compositions according to the present embodiment may also be those for using as therapeutic agents or prophylactic agents for obesity, dyslipidemia, impaired glucose tolerance, diabetes mellitus, arteriosclerosis, and inflammatory diseases. Further, the present embodiment may also be use of the compositions according to the present embodiment for producing pharmaceuticals for treating or preventing obesity, dyslipidemia, impaired glucose tolerance, diabetes mellitus, arteriosclerosis, and inflammatory diseases.

### (Embodiment 2)

Next, embodiment 2 of the present invention will be described.

The food and beverages according to this embodiment contains the above composition according to the embodiment 1. As described in Example 2 below, when orally administered such a composition brings about various effects and therefore can be used as a raw material for the food and beverages.

A form of the food and beverages when the above composition is used as the raw material of the food and beverages is, for example, beverages, such as nutritional energy drinks, soft drinks, tea, or green tea; confections, such as candies, cookies, tablet candies, chewing gums, or jellies; cereal processed products, such as noodles, breads, cooked rice, or biscuits; sausages; ham; fish paste products, such as kamaboko fish cake, dairy products, such as butter or yogurts; furikake rice seasonings; seasonings; or the like. It is to be noted that additives, such as sweeteners, flavors, or coloring agents may be contained in such food and beverages.

It is suitable that the food and beverages according to the present embodiment is eaten or drunk for the purpose of improvement of insulin resistance because the food and beverages contain the above composition which has effects of suppressing an increase in blood glucose level, weight gain, and lipid absorption. In addition, such food and beverages may be eaten or drunk for the purpose of treating or preventing diseases selected from, for example, obesity, dyslipidemia, impaired glucose tolerance, diabetes mellitus, and arteriosclerosis. Such food and beverages suppress ER stress and therefore may be eaten or drunk for the purpose of suppressing the ER stress, or treating or preventing diseases associated with the ER stress. Besides, such food and beverages may be eaten or drunk for the purpose of treating or preventing diseases such as coronary artery diseases, hypertension, metabolic syndrome, neurodegenerating disease, or bipolar disorder.

In addition, it is suitable that the food and beverages according to the present embodiment is used for the purpose of suppressing the IL-8 secretion from intestinal epithelial cells because the food and beverages contain the above composition that suppresses the IL-8 secretion from intestinal epithelial cells. Further, the food and beverages according to the present embodiment may also be eaten or drunk for the purpose of treating or preventing inflammatory diseases.

The content of the above composition as an active component in the food and beverages is usually 0.0001 to 100% by weight, and preferably 1 to 95% by weight. The amount of the above composition taken as an active component only need to be in a range of 1 mg to 100 g, preferably 10 mg to 100 g, and more preferably 100 mg to 50 g per adult per day.

As described in detail above, the food and beverages according to the present embodiment contains the γ-oryzanol incorporated in the biocompatible particle according to the above embodiment 1. Because of this, γ-oryzanol can be efficiently taken up into the body by taking such food and beverages. In addition, processing as food and beverages allows tastes, smells, flavors, or the like to be added in accordance with preference. Further, processing as food and beverages allows the γ-oryzanol incorporated in the biocompatible particle to be easily taken through meals.

### Examples

By way of the following examples, the present invention will be further specifically described; but the present invention is not limited by the examples.

### Example 1: Preparation of γ-oryzanol-enclosing particles

PLGA particles that enclosed γ-oryzanol were prepared. Two grams of PLGA (PLGA7520, lactic acid: glycolic acid = 75 : 25, weight average molecular weight: 20,000, manufactured by Wako Pure Chemical Industries, Ltd.) and 0.1 g of γ-oryzanol (manufactured by Wako Pure Chemical Industries, Ltd.) were dissolved in a mixed solvent of 40 ml of acetone and 20 ml of ethanol to obtain a polymer solution. This was dropped to 120 ml of 0.5% by weight polyvinyl alcohol solution (Gohsenol (trademark) EG50, manufactured by The Nippon Synthetic Chemical Industry Co., Ltd.) stirred at 40°C and 400 rpm at a constant rate (4 ml/min) to obtain a γ-oryzanol-incorporating particle suspension. Subsequently, while the suspension was stirred at 40°C and 100 rpm under reduced pressure, the mixed solvent was evaporated. After about two-hour evaporation of the mixed solvent distillation, the suspension was filtered (pore size; 32 µm); and the filtrate was subjected to freeze-drying overnight to obtain dried powder of γ-oryzanol-incorporating particles. The obtained dried powder has a number mean particle size of 198 nm and exhibits an incorporating percentage of γ-oryzanol of 3.49 ± 0.11 %(w/v) based on PLGA. The zeta potential of the γ-oryzanol-incorporating particle was 21.0 mV.

Here, the definition of the number mean particle size was defined as follows. A cumulative curve was determined with the total volume of the population of the powder as 100%, 50% diameter (D50) was defined as a parameter for generally evaluating particle size distribution as a cumulative median diameter (mean particle size), which 50% diameter was a particle size at a point where the cumulative curve reached 50%. The mean particle size was measured by subjecting a sample prepared by suspending nanoparticles in distilled water to a light scattering method using Microtrack UPA150 (manufactured by Nikkiso Co., Ltd.).

### Example 2: Evaluation of γ-oryzanol-incorporating particles in genetically obese mice (1)

The γ-oryzanol- incorporating particle prepared in Example 1 was orally administered to mice for evaluation.

First, the γ-oryzanol-incorporating particle was suspended in distilled water at 5%, 1.25%, and 0.31% (w/v). As a control, γ-oryzanol was suspended in 0.5% (w/v) methylcellulose solution (manufactured by Wako Pure Chemical Industries, Ltd.) at 0.05% and 3.2% (0.5 and 32 mg/ml).

Subsequently, each of the γ-oryzanol-incorporating particle suspensions and the γ-oryzanol suspensions was orally administered to ob/ob mice (five weeks old, male, B6. Lep^{ob}/J mice (homozygote (Lep^{ob}/Lep^{ob})), made by Charles River Laboratories Japan, Inc.) (n = 6) using a probe. For the details, the γ-oryzanol-incorporating particle suspension at 0.3, 1.3, and 5 µg/g of body weight/14 days in terms of γ-oryzanol was administered to the mice for two days followed by a five-day rest period every two weeks; and observation was made for 14 days. The daily dose was 1.1, 4.4, and 17.5µg/g of body weight/day, respectively. On the other hand, the γ-oryzanol suspension was orally administered daily to mice at 5 and 320 µg/g of body weight/day for 28 days in a raw; and observation was made for four weeks.

One week and two weeks after the beginning of the administration, the body weight and blood glucose level of the mice were measured. For the measurement of the blood glucose level, a simple blood glucose meter (MediSafe (registered trademark) Mini; manufactured by Terumo Corporation) was used. In addition, for the purpose of evaluating glucose tolerance, an oral glucose tolerance test was carried out ten days after the beginning of the administration. In such a test, following to 18-hour fasting, glucose was orally administered at 0.75 g/kg using a probe; and the blood glucose level was measured from the time of administration to two hours thereafter.

With regard to the mice administered with the γ-oryzanol-incorporating particle, triglyceride and insulin in the plasma and lipids in feces were quantified for the group administered with 5 µg/g of body weight/day; and, for the purpose of evaluating ER stress in β cells, the blood, feces, and the islets of Langerhans were collected from the mice two weeks after the beginning of the administration. The triglyceride in the plasma were measured using Triglyceride E-Test Wako (manufactured by Wako Pure Chemical Industries, Ltd.). The lipids in the feces were extracted by Folch method. It is to be noted that the quantification of the lipids in the feces was carried out in n = 2. The concentration of insulin in the plasma was measured using Ultra Sensitive Mouse Insulin ELISA Kit (manufactured by Morinaga Institute of Biological Science, Inc.).

As for the evaluation of ER stress in β cells, the expression level of ER stress marker genes (Chop, ERdj4, and Xbpls) was analyzed. First, total RNA in the islets of Langerhans was extracted using TRIzol (registered trademark) RNA Isolation Reagents (manufactured by Life Technologies Corporation); and cDNA was synthesized using iScript (trademark) cDNA Synthesis Kit (manufactured by Bio-Rad Laboratories, Inc.). Subsequently, using StepOnePlus (trademark) Real-Time PCR Systems and Fast SYBR (registered trademark) Green Master Mix (manufactured by Life Technologies Corporation). The expression level of ER stress marker genes was analyzed from the synthesized cDNA.

### (Results)

The body weight of the mice after the administration of the γ-oryzanol-incorporating particle is shown FIG. 1A. In the mice administered with the γ-oryzanol-incorporating particle, weight gain two weeks after the administration was significantly inhibited at any dose, as compared with a vehicle (p < 0.05 or 0.01). On the other hand, as shown in FIG. 1B, the significant inhibition of the weight gain was not observed in the mice administered with the γ-oryzanol.

The blood glucose level of the mice after the administration of the γ-oryzanol-incorporating particle is shown in FIG. 2A. In the mice administered with the γ-oryzanol-incorporating particle, the blood glucose level one week (p < 0.05) and two weeks (p < 0.01) after the administration significantly decreased in the group administered with 1.3 µg/g of body weight, as compared with the vehicle. In addition, in the group administered with 5 µg/g of body weight and the group administered with 0.3 µg/g of body weight, the blood glucose level two weeks after the administration was significantly decreased, as compared with the vehicle (p < 0.01). On the other hand, as shown in FIG. 2B, the significant decrease in the blood glucose level was not observed in the mice administered with the γ-oryzanol.

The results of the oral glucose tolerance test were shown in FIG. 3A, FIG. 3B, FIG. 3C and FIG. 3D. As shown in FIG. 3A, in the mice administered with the γ-oryzanol-incorporating particle, the blood glucose level 15 and 120 minutes after the glucose injection was significantly decreased in the group administered with 0.3 µg/g of body weight, as compared with the vehicle. In the group administered with 1.3 µg/g of body weight and the group administered with 5 µg/g of body weight, the blood glucose level 15, 30, and 120 minutes after the glucose injection were significantly decreased, as compared with the vehicle. FIG. 3B shows AUC for 120 minutes after the glucose injection. AUC was smaller in all of the groups of the mice administered with γ-oryzanol-incorporating particle, as compared with the vehicle. On the other hand, the mice administered with γ-oryzanol showed a comparable blood glucose level, as compared with the vehicle (FIG. 3C and FIG. 3D) and a decrease in the blood glucose level was not observed.

FIG. 4A shows the concentration of triglyceride in the plasma in the mice administered with the γ-oryzanol-incorporating particle (the group administered with 5 µg/g of body weight). It was confirmed that the concentration of triglyceride in the plasma tended to decrease, as compared with the vehicle. FIG. 4B shows the amount of fecal lipids in the mice administered with the γ-oryzanol-incorporating particle (the group administered with 5 µg/g of body weight). It was confirmed that the amount of lipids in the feces tended to increase, as compared with the vehicle. From these results, it was suggested that administration of the γ-oryzanol-incorporating particle inhibited the lipid absorption in the mice.

FIG. 5 shows the concentration of insulin in the plasma in the mice administered with the γ-oryzanol-incorporating particle (the group administered with 5 µg/g of body weight). When the γ-oryzanol-incorporating particle was administered, little change in the concentration of insulin was observed. Considering that ob/ob mice were model mice that exhibit insulin resistance and the blood glucose level significantly decreased in the mice administered with the γ-oryzanol-incorporating particle (see FIG. 2A), it was suggested that the γ-oryzanol-incorporating particle improved insulin resistance.

FIG. 6A, FIG. 6B and FIG. 6C show the expression level of ER stress marker genes (Chop, ERdj4, and Xbp1s) in the mice administered with the γ-oryzanol-incorporating particle (the group administered with 5 µg/g of body weight), compared with the vehicle. It was confirmed that the expression levels of Chop, ERdj4, and Xbp1s all tended to decrease. It was thereby suggested that the γ-oryzanol-incorporating particle had the effect of suppressing ER stress in β cells.

### Example 3: Evaluation of γ-oryzanol-incorporating particle's effect of suppressing IL-8 secretion upon TNF-α stimulation using human intestinal epithelial cell line

### (Preparation of cells)

As for cells, Caco-2 (RCB0988, RIKEN, the Institute of Physical and Chemical Research) was used. Caco-2 was cultured in a 100-ml culture dish until reaching semiconfluent. As for a medium, Dulbecco modified Eagle's Medium (DMEM, D5796, manufactured by Sigma-Aldrich) was used. To the medium, non-essential amino acids (M7145, manufactured by Sigma-Aldrich) and Penicillin/Streptomycin (hereinafter referred to as "PS"; manufactured by Gibco, Life Technologies Corporation Japan) were added.

Cells with 50 passages were detached with 0.25% trypsin/0.02% EDTA and seeded in a 24-well plate at a concentration of 2 × 10⁵ cells/well. The cells were cultured in DMEM with 10% FBS (fetal bovine serum), 1% non-essential amino acids, and 1% PS for 14 days. The medium was changed every other day; and the cells were sufficiently differentiated to be used the experiment below.

### (Pretreatment)

The medium was removed 24 hours before stimulation with TNF-α; and the cells were washed with 1 ml of PBS per well twice. Subsequently, the medium in each well was replaced with 1 ml of 1% PS-containing DMEM added with γ-oryzanol, the γ-oryzanol-incorporating particle, or the FITC-incorporating particle, which was prepared at the time of use; and the cells were cultured for 24 hours. Here, the used medium containing γ-oryzanol, the γ-oryzanol-incorporating particle, or the FITC-incorporating particle was prepared as follows.

With regard to γ-oryzanol, γ-oryzanol (152-01272, manufactured by Wako Pure Chemical Industries, Ltd.) was dissolved in acetone at 10 mg/ml, serially diluted with acetone, and then added to the medium (the final concentration of acetone was 0.1%).

With regard to the γ-oryzanol-incorporating particle, the γ-oryzanol-incorporating particle with an incorporating percentage of 3.18 ±0.06% (w/v) and a number mean particle size of 220 nm, which particle was prepared in the same manner as described in Example 1, was used. The γ-oryzanol-incorporating particle was prepared with sterilized water at 15.72 mg/ml in term of PLGA (corresponding to 500 µg of bulk powder/ml). The resultant was further diluted with the medium to 3.14 mg/ml (corresponding to 100 µg of bulk powder/ml), which was serially diluted 10 fold with the medium.

With regard to the FITC (fluorescein isothiocyanate) incorporating particle, the FITC-incorporating particle with an incorporating percentage of 4.06% (w/v) and a number mean particle size of 225 nm, which particle was prepared in the same manner as described in Example 1, was prepared with the medium in the same manner as described for the above γ-oryzanol-enclosing particle.

It is to be noted that the medium used in the pretreatment was prepared at the time of use.

### (Stimulation with TNF-α)

The medium for the pretreatment was removed and thereafter the cells were washed with PBS twice. Subsequently, DMEM containing 1% FBS and 1% PS added with TNF-α (T6674-10UG, manufactured by Sigma-Aldrich) at 50 ng/ml or 100 ng/ml was added to each well; and the cells cultured for 24 hours. After culturing for 24 hours, the culture supernatant was collected in a 1.5 ml tube. It is to be noted that TNF-α that had dissolved in distilled water at 100 µg/ml and stored at -20°C was used. It is also to be noted that the medium was prepared at the time of use.

### (Quantification of IL-8)

The collected supernatant was subjected to ELISA (enzyme immunity measurement assay) to measure the amount of IL-8 in the supernatant. The measurement was carried out according to a protocol of an ELISA kit (D8000C, manufactured by R&D). At the same time, the total amount of proteins in the supernatant was measured by using a bicinchoninic acid assay (Pierce BCA Protein Assay Kit, manufactured by Thermo Scientific). For statistical analysis of data, one-way ANOVA Dunnett's Multiple Comparison test was employed.

### (Results)

FIG. 7A, FIG. 7B and FIG. 7C show values obtained by correcting the amount of IL-8 measured by ELISA with the total amount of proteins. FIG. 7A shows the amount of IL-8 in the supernatant when the cells were pretreated with γ-oryzanol. A decrease in the amount of IL-8 was observed at 1 µg/ml of γ-oryzanol but there was no significant difference, as compared with no (0 µl/ml) γ-oryzanol and 0.1 µg/ml of γ-oryzanol.

FIG. 7B shows the amount of IL-8 in the supernatant when the cells were pretreated with the γ-oryzanol-incorporating particle. A significant decrease of the amount of IL-8 was observed at an amount equivalent to 1 µg/ml of γ-oryzanol bulk powder, as compared with no γ-oryzanol (0µl/ml).

FIG. 7C shows the amount of IL-8 in the supernatant when the cells were pretreated with the FITC-incorporating particle which was used as a control against the PLGA particle. No significant difference was observed at any concentration, as compared with no FITC-incorporating particles.

The amount of IL-8 produced increases in intestinal epithelial cells stimulated with various factors. Increased secretion of IL-8 leads to recruitment of immune cells such as neutrophils; and the recruited immune cells release a large amount of inflammatory cytokines to thereby advance inflammation. From the results of the present example, the γ-oryzanol-incorporating particle has an excellent anti-inflammatory effect in intestinal epithelial cells.

### Example 4: Evaluation of uptake of FITC-incorporating particles into human intestinal epithelial cells

### (Preparation of cells)

Caco-2 used in the above Example 3 was cultured in the same manner.

Cells with 49 passages were detached with 0.25% trypsin/0.02% EDTA and seeded in a 35 mm glass (D110400, manufactured by Matsunami Glass Ind., Ltd.) at a concentration of 3 × 10⁵ cells/well. The cells were cultured in DMEM with 10% FBS, 1% non-essential amino acids, and 1% PS for 21 days. The medium was changed every other day and the cells were sufficiently differentiated to be used the experiment below.

### (Pretreatment)

The medium was removed 30 minutes before addition of FITC-incorporating particles; and the cells were washed with 2 ml of PBS per well twice. Subsequently, the medium in each well was replaced with 1% PS-containing DMEM (without serum). Next, the medium was 2 ml of 1% PS-containing DMEM added with the FITC-incorporating particle or FITC; and the cells were incubated at 37°C for two hours. The medium used here containing the FITC-incorporating particle and FITC was prepared as follows.

With regard to the FITC-incorporating particle, the FITC-incorporating particle with an incorporating percentage of 4.06% (w/v) and a number mean particle size of 225 nm, which particle was prepared in the same manner as described in Example 1, was dissolved in water to prepare a 1 mM (9.59 mg/ml) aqueous solution, which was diluted with 1% PS-containing DMEM. The concentration of particles was 0.0959 mg/ml (10 µM in terms of FITC).

With regard to FITC, FITC bulk powder (FITC-1 343-03664, manufactured by Dojindo Laboratories) was prepared to be 10 mM with 0.1% DMSO (dimethyl sulfoxide) and diluted with 1% PS-containing DMEM.

### (Preparation of samples and evaluation thereof by confocal laser scanning microscope)

After the above two-hour incubation, 7.5 µM cellMask (trademark) and 1% PS-containing DMEM were added at 2 ml/well, followed by incubation at 37°C and washing with HBSS buffer (Gibco 14175-095, manufactured with Life Technologies Corporation Japan) for five minutes three times. And then, the cells were fixed with chilled methanol, washed with PBS, and then mounted with DAPI, thereby obtaining a sample zero days (two hours) after the beginning of the incubation.

After washed with PBS, the cells were cultured in DMEM medium with 10% FBS, 1% non-essential amino acids, and 1% PS (the medium was changed every other day) and fixed at each time point of one, three, seven days after the beginning of the incubation in the above pretreatment. Before the fixation, 7.5 µM cellMask (trademark) and 1% PS-containing DMEM were added at 2 ml/well, followed by incubation at 37°C and washing with HBSS buffer for five minutes three times. Subsequently, because cellMask (trademark), which stains the plasma membrane, solved out upon the fixation with methanol, the cells were fixed with formalin, incubated with PBS containing 0.1% triton-X for 10 minutes, washed with PBS, and mounted with DAPI, thereby obtaining samples one, three, seven days after the beginning of the incubation.

Each of the samples obtained as described above was photographed by a confocal laser scanning microscope (Nikon confocal microscope system A1+, manufactured by Nikon Corporation); and evaluated for fluorescence intensity at each concentrations.

### (Results)

FIG. 8A, FIG. 8B, FIG. 8C and FIG. 8D show the average value of the fluorescence intensity of the FITC-incorporating particle and FITC which photographed by the confocal laser scanning microscope in the samples zero (two hours), one, three, seven days after the beginning of the incubation, respectively. It was shown that more FITC-incorporating particles were taken up into cells at least for seven days or more after the exposure to the cells, as compared with FITC which was not incorporated in PLGA.

Taken together, the γ-oryzanol-incorporating particle improved the insulin resistance and glucose metabolism in the obese mice that were orally administered with the γ-oryzanol-incorporating particle (0.3, 1.3, and 5 µg/g of body weight) for two days every two weeks. These effects were not observed at all in the obese mice that were orally administered with the γ-oryzanol that was not incorporated in the PLGA particle for 14 days (the total amount of γ-oryzanol administered was the same as that in the group administered with 5 µg/g of body weight). When compared with 14-day oral administration of γ-oryzanol (320 µg/g × 14 = 4480 µg/g) in the above Non Patent Literature 1, it turns out that the γ-oryzanol-incorporating particle in the present example brings about a comparable effect at 1/533 to 1/64 of dose. In addition, γ-oryzanol-incorporating particle's tendencies of improving the lipid metabolism and suppressing the ER stress were observed.

Further, the γ-oryzanol-incorporating particle suppressed the IL-8 secretion induced by the stimulation of intestinal epithelial cells with TNF-α. From this, it is suggested that the γ-oryzanol-incorporating particle is effective for inflammatory diseases such as ulcerative colitis and Crohn's disease which are associated with IL-8 in the intestinal epithelia; or the like.

From the result of increasing the uptake of the γ-oryzanol-incorporating PLGA particle into intestinal epithelial cells over at least seven days or more, it is thought that orally-administered γ-oryzanol-incorporating particle was distributed mainly to the intestinal tract, retained in the mucosa layer and the intestinal wall over seven days or more after the administration, and released γ-oryzanol, thereby resulting in various excellent effects exhibited in the above examples of the γ-oryzanol-incorporating particle. That is, the incorporation of γ-oryzanol in the PLGA particle presumably led to remarkable enhancement of the retention and absorption of γ-oryzanol in the intestinal tract, thereby producing exceptional effects at a low γ-oryzanol dose in the present example. It is to be noted that the uptake of the PLGA particles into cells was confirmed to be maintained over 14 days.

### Example 5: Evaluation of γ-oryzanol-incorporating particles in genetically obese mice (2)

### (Experimental animals)

Ob/ob mice were raised at 24°C under a light/dark cycle of 12 hours/12 hours. The mice were raised in conditions where they were able to freely take feed and water. The body weight of mice was measured every week.

### (Administration of γ-oryzanol)

γ-oryzanol incorporated with PLGA particles was dissolved in distilled water (hereinafter referred to as "Nano Orz") and administered to the mice using a probe at a dose of 0.3, 1.3, or 5 µg/g of body weight per day for two days every two weeks. As a solution of γ-oryzanol which was not enclosed in the PLGA particle, γ-oryzanol (manufactured by Wako Pure Chemical Industries, Ltd.) was dissolved in a 0.5% methylcellulose solution (hereinafter referred to as "Orz"). Orz was administered to the stomach using a probe everyday for four weeks (5 or 320 µg/g of body weight/day).

### (Evaluation of metabolism)

Whole blood was collected from the tail vein of mice; and blood glucose was measured using a simple blood glucose meter. A blood sample was further collected from the network of the retro orbital vein. For a glucose tolerance test, the mice were fasted for 18 hours and thereafter administered with glucose at 0.75 g/kg of body weight. The blood glucose level was measured at the predetermined time. In addition, for an insulin tolerance test, insulin (0.5 unit/kg of body weight; manufactured by Eli Lilly) was intraperitoneally injected to the mice that had fasted for four hours. The concentration of triglycerides in the plasma was determined by Triglyceride E-Test Wako.

### (Evaluation of ER stress and inflammation in tissues)

The hypothalamus, the islets of Langerhans, the adipose tissue of the mesentery, the liver, the epididymal adipose tissue, and the subcutaneous adipose tissue were collected from the mice; and the expression level of ER stress marker genes (Chop, ERdj4, and Xbp1s), and inflammation marker genes (TNF-α and MCP-1) was quantified using a quantitative real time polymerase chain reaction. The islets of Langerhans was isolated from mice by digestion with collagenase (Liberase TL; manufactured by Roche Diagnostics K.K.) and purified using Histopaque (trademark) gradient (Histopaque 1077; manufactured by Sigma-Aldrich).

Using TRIzol (registered trademark) RNA Isolation Reagents (manufactured by Life Technologies Corporation), total RNA was extracted from each of the tissues; and cDNA was synthesized using iScript (trademark) cDNA Synthesis Kit (manufactured by Bio-Rad Laboratories, Inc.). Next, quantitative real time PCR was carried out using StepOnePlus (trademark) Real-Time PCR System, Fast TaqMan Universal Master Mix, and Fast SYBR Green Master Mix (manufactured by Applied Biosystems). The level of mRNA was normalized with that of 18S rRNA.

### (Statistical analysis)

Data were expressed as mean ±standard error (s.e.m). One-way analysis of variance, repeated measures analysis of variance, and multiple comparisons following repeated measures analysis of variance (Bonferroni/Dunn method) were employed as appropriate. For analysis of difference between two groups, Student's t-test was employed. Difference was considered to be significant with P < 0.05.

### (Results)

The results of the present example are shown below. FIG. 9A and FIG. 9B show a change in the body weight of genetically obese mice in a time-dependent manner (n = 14). According to FIG. 9A, significant suppression of the weight gain was shown even at a very low dose in the genetically obese mice administered with Nano Orz which was incorporated in the PLGA particle. On the other hand, according to FIG. 9B, no significant suppression of the weight gain was observed even when the administration was performed at 320 µg/g of body weight/day in the genetically obese mice administered with Orz which was not incorporated in the PLGA particle.

FIG. 10A and FIG. 10B show a change in the blood glucose level of genetically obese mice in a time-dependent manner (n = 14). According to FIG. 10A, hyperglycemia was surely ameliorated even at about 1/100 of the maximum administration amount of Orz in the genetically obese mice administered with Nano Orz. On the other hand, according to FIG. 10B, no significant change in the blood glucose level was observed even when the administration was performed at 320 µg/g of body weight/day in the genetically obese mice administered with Orz. It was thus demonstrated that hyperglycemia in the genetically obese mice was prominently ameliorated by Nano Orz, as compared with Orz.

The results of the glucose tolerance test were shown in FIG. 11A, FIG. 11B, FIG. 11C and FIG. 11D (n = 14). According to FIG. 11A and FIG. 11B, a marked decrease in the glucose tolerance was prominently improved even at a low dose in the genetically obese mice administered with Nano Orz. On the other hand, according to FIG. 11C and FIG. 11D, no improvement of a marked decrease in the glucose tolerance was observed even when the administration was performed at 320 µg/g of body weight/day in the genetically obese mice administered with Orz.

The results of the insulin tolerance test were shown in FIG. 12A, FIG. 12B, FIG. 12C and FIG. 12D (n = 14). According to FIG. 12A and FIG. 12B, the insulin tolerance was significantly improved even at a low dose in the genetically obese mice administered with Nano Orz. On the other hand, according to FIG. 12C and FIG. 12D, no improvement of the insulin tolerance was observed even when the administration was performed at 320 µg/g of body weight/day in the genetically obese mice administered with Orz.

FIG. 13 shows the concentration of plasma triglyceride in the genetically obese mice (n = 6). While the concentration of triglyceride was significantly decreased even at a low dose in the case where Nano Orz was administered, the concentration of triglyceride was not decreased at the same amount of Orz administered.

FIG. 14A and FIG. 14B show the expression level of Chop and ERdj4 in the hypothalamus, respectively (N = 8). While Nono Orz significantly inhibited the expression level of Chop at a dose of 1.3 and 5 µg/g of body weight, Orz did not inhibit the expression level of Chop even when the administration was performed at 320 µg/g of body weight/day. In addition, while Nono Orz significantly inhibited the expression level of ERdj4 at a dose of 0.3, 1.3 and 5 µg/g of body weight, Orz did not inhibit the expression level of Chop even when the administration was performed at 320 µg/g of body weight/day.

FIG. 14C and FIG. 14D show the expression level of ERdj4 and Xbp1sin the islets of Langerhans, respectively (N = 8). While Nano Orz significantly inhibited the expression level of ERdj4 at a dose of 0.3, 1.3 and 5 µg/g of body weight, Orz did not inhibit the expression level of ERdj4 even when the administration was performed at 320 µg/g of body weight/day. In addition, while Nano Orz significantly inhibited the expression level of Xbp1s at a dose of 0.3 and 1.3 µg/g of body weight, Orz did not inhibit the expression level of Xbp1s even when the administration was performed at 320 µg/g of body weight/day.

FIG. 15A and FIG. 15B show the expression level of Chop and Xbp1s in the adipose tissue in the mesentery, respectively (N = 8). Orz significantly inhibited expression of Chop at 320 µg/g of body weight/day. On the other hand, Nano Orz significantly inhibited the expression level of Chop at a dose of 5 µg/g of body weight. In addition, while Nano Orz significantly inhibited the expression level of Xbp1s at a dose of 5 µg/g of body weight, Orz did not inhibit the expression level of Xbp1s even when the administration was performed at 320 µg/g of body weight/day.

FIG. 15C and FIG. 15D show the expression level of Chop and Xbp1s in the liver, respectively (N = 8). While Nano Orz significantly inhibited the expression level of Chop at a dose of 1.3 and 5 µg/g of body weight, Orz did not inhibit the expression level of ERdj4 even when the administration was performed at 320 µg/g of body weight/day. In addition, while Nano Orz significantly inhibited the expression level of Xbp1s at a dose of 0.3 and 1.3 µg/g of body weight, Orz did not inhibit the expression level of Xbp1s even when the administration was performed at 320 µg/g of body weight/day.

FIG. 16A and FIG. 16B show the expression level of TNF-α and MCP-1 in the epididymal adipose tissue, respectively (N = 8). While Nano Orz significantly inhibited the expression level of TNF-α at a dose of 0.3, 1.3 and 5 µg/g of body weight, Orz did not inhibit the expression level of TNF-α even when the administration was performed at 320 µg/g of body weight/day. In addition, while Nano Orz significantly inhibited the expression level of MCP-1 at a dose of 0.3 and 1.3 µg/g of body weight, Orz did not inhibit the expression level of MCP-1 even when the administration was performed at 320 µg/g of body weight/day.

FIG. 16C and FIG. 16D show the expression level of TNF-α and MCP-1 in the subcutaneous fatty tissue, respectively (N = 8). While Nano Orz significantly inhibited the expression level of TNF-αat a dose of 0.3, 1.3 and 5 µg/g of body weight, Orz did not inhibit the expression level of TNF-αeven when the administration was performed at 320 µg/g of body weight/day. In addition, while Nano Orz significantly inhibited the expression level of MCP-1 at a dose of 0.3, 1.3 and 5 µg/g of body weight, Orz did not inhibit the expression level of MCP-1 even when the administration was performed at 320 µg/g of body weight/day.

From the results of FIG. 14A, FIG. 14B, FIG. 14C and FIG. 14D, FIG. 15A, FIG. 15B, FIG. 15C and FIG. 15D, it was suggested that γ-oryzanol was not able to inhibit the ER stress in the hypothalamus, the islets of Langerhans, and the liver even when the administration was performed at 320 µg/g of body weight/day which was an overdose, whereas the γ-oryzanol incorporated in the PLGA particle was able to inhibit the ER stress in the hypothalamus, the islets of Langerhans, the fatty tissue of the mesentery, and the liver at a very low dose. In addition, from the results of FIG. 16A, FIG. 16B, FIG. 16C and FIG. 16D, it was suggested that γ-oryzanol was not able to inhibit the inflammation in the epididymal adipose tissue and the subcutaneous adipose tissue even when the administration was performed at 320 µg/g of body weight/day which was an overdose, whereas the γ-oryzanol incorporated in the PLGA particle was able to inhibit the inflammation in the epididymal adipose tissue and the subcutaneous adipose tissue at a very low dose.

### Industrial Applicability

The present disclosure is suitable to improvement of insulin resistance, treatment or prevention of obesity, dyslipidemia, impaired glucose tolerance, diabetes mellitus, arteriosclerosis, and inflammatory diseases including intestinal inflammatory diseases, such as ulcerative colitis or Crohn's disease. Application of the present disclosure improves results of treatment or prevention of those diseases and secures patients' quality of life.

## Claims

1. A composition containing
γ-oryzanol and
a biocompatible particle incorporating the γ-oryzanol in the inside thereof, wherein the biocompatible particle has a number mean particle size of 2.5 to 1,000 nm and comprises a polylactideglycolide copolymer or a polyethylene glycol modification of the polylactideglycolide copolymer.

2. The composition according to claim 1, for use as a medicament.

3. The composition for use according to claim 2, for use in improvement of insulin resistance.

4. The composition for use according to claim 2 or 3, for use in suppression of endoplasmic reticulum stress.

5. The composition for use according to any one of claims 2-4, for use in suppression of IL-8 secretion from an intestinal epithelial cell.

6. The composition for use according to any one of claims 2-5, for use in treatment or prevention of a disease selected from the group consisting of obesity, dyslipidemia, impaired glucose tolerance, diabetes mellitus, arteriosclerosis, and inflammatory diseases.

7. The composition for use according to any one of claims 2-6, wherein the composition is administered to a patient with a dosing interval of one day or more.

8. The composition for use according to claim 7, wherein the dosing interval is seven days or more and 14 days or less.

9. A food or beverage comprising the composition according to claim 1.

10. The composition for use according to any of claims 2-8, wherein the composition is administered as food or beverage.

## Patentansprüche

1. Zusammensetzung enthaltend
γ-Oryzanol und
ein biokompatibles Teilchen, inkorporierend das γ-Oryzanol in dem Inneren davon, wobei das biokompatible Teilchen eine zahlenmittlere Teilchengröße von 2,5 bis 1.000 nm hat und ein Polylactidglycolid-Copolymer oder eine Polyethylenglycol-Modifikation des Polylactidglycolid-Copolymers umfasst.

2. Zusammensetzung nach Anspruch 1, zur Verwendung als ein Arzneimittel.

3. Zusammensetzung zur Verwendung nach Anspruch 2, zur Verwendung bei Verbesserung von Insulinresistenz.

4. Zusammensetzung zur Verwendung nach Anspruch 2 oder 3, zur Verwendung bei Unterdrückung von endoplasmatischem Retikulumstress.

5. Zusammensetzung zur Verwendung nach einem der Ansprüche 2 - 4, zur Verwendung bei Unterdrückung von IL-8-Sekretion aus einer Darmepithelzelle.

6. Zusammensetzung zur Verwendung nach einem der Ansprüche 2 - 5 zur Verwendung bei Behandlung oder Prävention einer Krankheit, ausgewählt aus der Gruppe bestehend aus Fettleibigkeit, Dyslipidämie, gestörter Glukosetoleranz, Diabetes mellitus, Arteriosklerose, und entzündlichen Krankheiten.

7. Zusammensetzung zur Verwendung nach einem der Ansprüche 2 - 6, wobei die Zusammensetzung einem Patienten mit einem Dosierungsintervall von einem Tag oder mehr verabreicht wird.

8. Zusammensetzung zur Verwendung nach Anspruch 7, wobei das Dosierungsintervall sieben Tage oder mehr und 14 Tage oder weniger ist.

9. Nahrungsmittel oder Getränk, umfassend die Zusammensetzung nach Anspruch 1.

10. Zusammensetzung zur Verwendung nach einem der Ansprüche 2 - 8, wobei die Zusammensetzung als Nahrungsmittel oder Getränk verabreicht wird.

## Revendications

1. Composition contenant :
du γ-oryzanol et
une particule biocompatible incorporant le γ-oryzanol dans l'intérieur de celle-ci, dans laquelle la particule biocompatible présente une taille de particule moyenne en nombre de 2,5 à 1 000 nm et comprend un copolymère de polylactideglycolide ou une modification par du polyéthylène glycol du copolymère de polylactideglycolide.

2. Composition selon la revendication 1, pour une utilisation comme un médicament.

3. Composition pour une utilisation selon la revendication 2, pour une utilisation dans l'amélioration de la résistance à l'insuline.

4. Composition pour une utilisation selon la revendication 2 ou 3, pour une utilisation dans la suppression de stress du réticulum endoplasmique.

5. Composition pour une utilisation selon l'une quelconque des revendications 2-4, pour une utilisation dans la suppression de sécrétion IL-8 d'une cellule épithéliale intestinale.

6. Composition pour une utilisation selon l'une quelconque des revendications 2-5, pour une utilisation dans le traitement ou la prévention d'une maladie choisie dans le groupe consistant en obésité, dyslipidémie, tolérance altérée au glucose, diabètes sucrés, artériosclérose, et maladies inflammatoires.

7. Composition pour une utilisation selon l'une quelconque des revendications 2-6, dans laquelle la composition est administrée à un patient avec un intervalle de dosage d'une journée ou plus.

8. Composition pour une utilisation selon la revendication 7, dans laquelle l'intervalle de dosage est de sept jours ou plus et de 14 jours ou moins.

9. Aliment ou boisson comprenant la composition selon la revendication 1.

10. Composition pour une utilisation selon l'une quelconque des revendications 2-8, dans laquelle la composition est administrée comme aliment ou boisson.
